# EUROPEAN PATENT SPECIFICATION

(11) **EP 3 438 242 B1**
(45) Date of publication and mention of the grant of the patent: **17.02.2021**
(21) Application number: 17773815.0
(22) Date of filing: 17.02.2017
(51) Int. Cl.: C12M 1/34, C12Q 1/02, C12N 5/078, C12N 5/09, G01N 33/50

(54) **CELL OBSERVATION DEVICE, METHOD FOR EVALUATING ACTIVITY LEVEL OF IMMUNE CELLS, AND METHOD FOR CONTROLLING QUALITY OF IMMUNE CELLS**
ZELLBEOBACHTUNGSVORRICHTUNG, VERFAHREN ZUR BEWERTUNG DES AKTIVITÄTSNIVEAUS VON IMMUNZELLEN UND VERFAHREN ZUR KONTROLLE DER QUALITÄT VON IMMUNZELLEN
DISPOSITIF D'OBSERVATION DE CELLULES, PROCÉDÉ D'ÉVALUATION DU NIVEAU D'ACTIVITÉ DE CELLULES IMMUNITAIRES, ET PROCÉDÉ DE RÉGULATION DE LA QUALITÉ DE CELLULES IMMUNITAIRES

(30) Priority: 30.03.2016 JP 2016068641
(43) Date of publication of application: 06.02.2019
(73) Proprietor: Sony Corporation, Tokyo 108-0075 (JP)
(72) Inventor: NAKAMURA Tomohiko, Tokyo 108-0075 (JP); BRUN Marcaurele, Tokyo 108-0075 (JP)
(74) Representative: D Young & Co LLP
(86) International application number: PCT/JP2017/005926
(87) International publication number: WO 2017/169267

(56) References cited:
- WO-A1-2009/110614
- JP-A- 2005 506 083
- JP-A- 2005 521 425
- JP-A- 2012 515 548
- TAE-JIN KIM ET AL: "Homotypic NK cell-to-cell communication controls cytokine responsiveness of innate immune NK cells", SCIENTIFIC REPORTS, vol. 4, no. 1, 5 December 2014 (2014-12-05), XP55531139, DOI: 10.1038/srep07157
- PER E. OLOFSSON ET AL: "Distinct Migration and Contact Dynamics of Resting and IL-2-Activated Human Natural Killer Cells", FRONTIERS IN IMMUNOLOGY, vol. 5, 1 March 2014 (2014-03-01), XP055531058, DOI: 10.3389/fimmu.2014.00080
- MARTIN WIKLUND ET AL: "Ultrasound-Induced Cell-Cell Interaction Studies in a Multi-Well Microplate", MICROMACHINES, vol. 5, no. 1, 6 February 2014 (2014-02-06), pages 27-49, XP055531046, DOI: 10.3390/mi5010027
- JOHANNA TAURIAINEN ET AL: "Single-Cell Characterization of in vitro Migration and Interaction Dynamics of T Cells Expanded with IL-2 and IL-7", FRONTIERS IN IMMUNOLOGY, vol. 6, 1 January 2015 (2015-01-01), page 196, XP055530785, DOI: 10.3389/fimmu.2015.00196
- Cristian Payes ET AL: "Cell Interaction Analysis by Imaging Flow Cytometry" In: "Cell Interaction", 10 October 2012 (2012-10-10), InTech, XP055530739, ISBN: 978-953-51-0792-7 DOI: 10.5772/51147, * the whole document * * In particular: Title; Abstract; Pages 306-314, Sections 2.2 and 3. *
- TAKAO HAYAKAWA: 'Science of Evaluating the Quality and Safety of Biotechnological Products' PDA JOURNAL OF GMP AND VALIDATION IN JAPAN, [Online] vol. 3, no. 2, 2001, ISSN 1881-1728 pages 57 - 66, XP055508348 ISSN: 1881-1728 Retrieved from the Internet: <URL:https://doi.org/10.11347/pda.3.57>

## Description

### [Technical Field]

The present invention relates to a cell observation device, a method for evaluating activity level of immune cells, and a method for controlling quality of immune cells.

### [Background Art]

In the past, in a cytotoxicity assay related to a plurality of kinds of cells, a technique using a radioactive isotope (Cr51 release assay) has been used. This approach, however, has been limited in regard of operation and has required complicated operations. In addition, it is necessary to once cause Cr51 to be taken into the cell to be injured, and, the degree thereof varies depending on the kind of the cells, which has been a cause of variability of measurement accuracy.

In a Lactate Dehydrogenase (LDH) Assay, which is a technique not using a radioactive isotope, injury on cells can be measured by measuring the lactate dehydrogenase (LDH) released from the cells. According to this technique, however, it is impossible to discriminate which of target cells and effector cells are relevant to the injury that can be being measured, and the background noise is high. In addition, only a signal change on a bulk basis is measured, and it has been impossible to measure how many target cells are being injured by each of the individual effector cells.

Meanwhile, in recent years, an immunotherapy for treating cancer has been being established, and grown immune cells with high quality have been demanded. Immune cells injure or prey on cancer cells, but the injuring property thereof is evaluated only qualitatively (NPL 1).

In addition, a cell contact assay in which immune cells and cancer cells are brought into contact with each other to injure the cancer cells and the result is evaluated through fluorescence has been conducted (NPL 2). However, there has not been disclosed an assay by which to specifically determined how many cancer cells are injured by one immune cell.

Tae-Jin Kim et al., "Homotypic NK cell-to-cell communication controls cytokine responsiveness of innate immune NK cells", Scientific Reports, vol. 4, no. 1, 5 December 2014, demonstrate, both in vitro and in vivo, that innate immune NK cells can engage in homotypic NK-to-NK cell interactions for optimal survival, activation and proliferation.

Per E. Olofsson at al.: "Distinct Migration and Contact Dynamics of Resting and IL-2-Activated Human Natural Killer Cells", Frontiers in Immunology, vol. 5, 1 March 2014 present experiments that quantify, at the single-cell level, how activation by IL-2 leads to altered NK cell cytotoxicity, migration behaviour and contact dynamics.

Martin Wiklund et al., "Ultrasound-Induced Cell-Cell Interaction Studies in a Multi-Well Microplate", Micromachines, vol. 5, no.1, 6 February 2014, describe the use of ultrasound for inducing and retaining cell-cell contact in multi-well microplates combined with live-cell fluorescence microscopy.

Johanna Taurianen et al., "Single-Cell Characterization of in vitro Migration and Interaction Dynamics of T Cells Expanded with IL-2 and IL-7", Frontiers in Immunology, vol. 6, 1 January 2016, page 196, describe use of microchip-based live-cell imaging to follow the migration of individual T cells.

Christian Payes et al., "Cell Interaction Analysis by Imaging Flow Cytometry", In: "Cell Interaction", 10 October 2012, InTech, describe using Multispectral Imaging Flow Cytometry (MIFC) to analyse cell-cell interaction between cells belonging to the same species.

### [Citation List]

### [Non Patent Literature]

[NPL 1]
   Immunity. 2015 May 19, 42(5): 864-76
[NPL 2]
   Bioinformatics. 2015 Oct 1; 31(19): 3189-97

### [Summary]

### [Technical Problem]

In view of the foregoing, the present inventors made extensive and intensive studies, and have completed a method for quantitatively evaluating the activity of cells on an individual cell basis, instead of a bulk basis, and a device to be used therefor.

### [Solution to Problem]

Specifically, the present technology provides:
a method for evaluating activity level of immune cells, comprising:
bringing a plurality of cancer cells into contact with a single immune cell; and
examining the number of cancer cells brought to death by the single immune cell within a predetermined time.

The present technology further provides a cell observation device for carrying out the above method, the apparatus comprising:
a cell introduction section;
a cell arrangement section comprising a plurality of wells;
an observation section for obtaining image data of cells within the cell arrangement section; and
an analysis section for analyzing the image data,
wherein the cell introduction section is configured to introduce immune cells and cancer cells into the cell arrangement section,
wherein one well of the cell arrangement section is arranged to contain a single immune cell and a plurality of cancer cells at an initial time,
the observation section is configured to make an observation of a temporal event resulting from cell contact in the one well of the cell arrangement section, and
the analysis section is configured to analyze the temporal event resulting from the cell contact to examine the number of the cancer cells brought to death by the single immune cell within a predetermined time of the initial time.

The present technology further provides a method for controlling quality of immune cells, comprising:
bringing a plurality of cancer cells into contact with a single immune cell;
examining the number of cancer cells brought to death by the single immune cell; and
excluding the single immune cell in a case where the single immune cell has brought a number of less than a predetermined number of the cancer cells to death or in a case where the single immune cell has brought a number of more than a predetermined number of the cancer cells to death within a predetermined time.

### [Advantageous Effects of Invention]

According to the present technology, cell injury or preying-on or the like by a single cell can be observed, the activity of the single cell can be quantitatively evaluated, and a cell suitable for use can be selected on the basis of the evaluation.

Note that the effects described here are not limitative, and any of the effects described herein may be provided or used.

### [Brief Description of Drawings]

[FIG. 1]

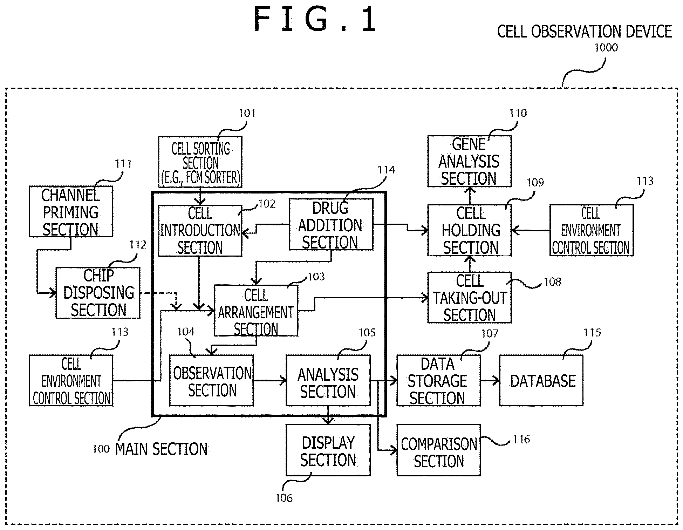

   FIG. 1 is a schematic diagram of a cell observation device according to the present technology.
[FIG. 2]

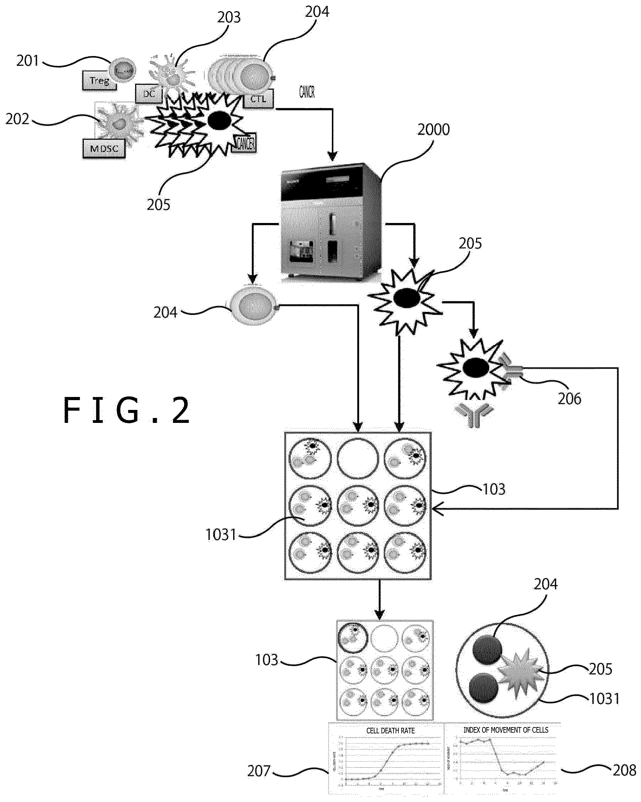

   FIG. 2 is a schematic figure depicting an example of cell observation and analysis according to the present technology.
[FIG. 3]

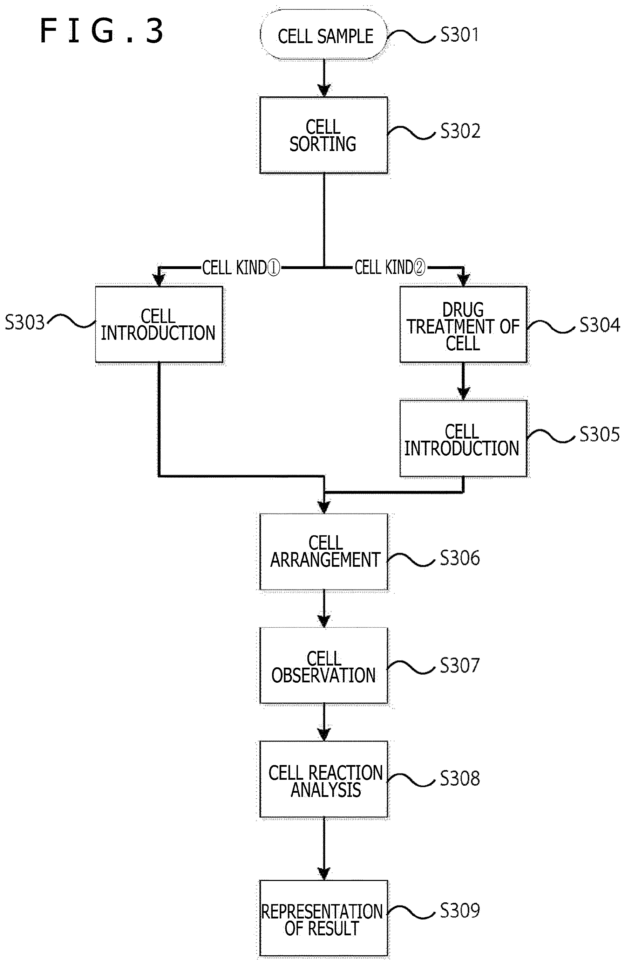

   FIG. 3 is a flow chart depicting an example of cell observation and analysis according to the present technology.
[FIG. 4]

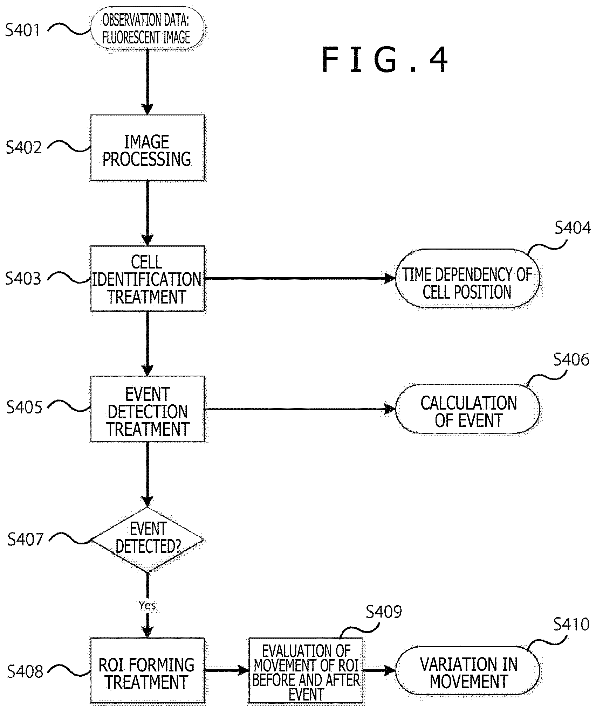

   FIG. 4 is a flow chart depicting an operation of an observation section of the cell observation device according to the present technology.
[FIG. 5]

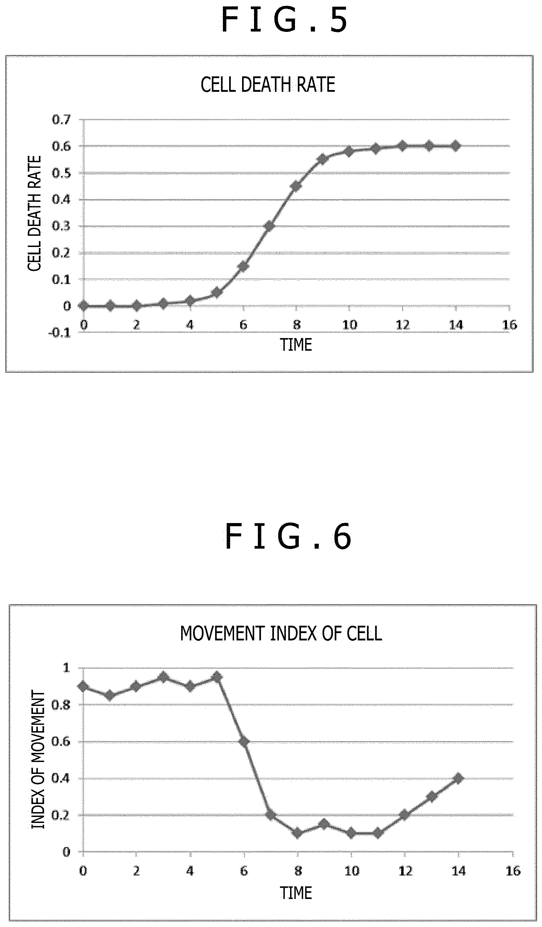

   FIG. 5 is a simulated graph of cancer cell death rate with the lapse of time.
[FIG. 6]
   FIG. 6 is a simulated graph depicting activeness of variation in movement of cytotoxic T cells.

### [Description of Embodiments]

Preferred embodiments for carrying out the present technology will be described below. Note that the following embodiments depict typical embodiments of the present technology, and the scope of the present technology is not to be construed narrowly thereby. The descriptions will be made in the following order.
1. Cell observation device
   1-1. Configuration of device
   1-2. Example of cell observation and analysis
   1-3. Data analysis
2. Method for evaluating activity level of immune cells
   2-1. Evaluation by cytotoxicity assay
   2-2. Evaluation by secretion release assay
   2-3. Evaluation by antibody-dependent cellular cytotoxicity
   2-4. Application example 1
   2-5. Application example 2
3. Method for controlling quality of immune cells

### <1. Cell observation device>

### 1-1. Configuration of device

An example of a cell observation device according to the present technology is schematically depicted in FIG. 1.

The cell observation device of the present technology includes at least a cell introduction section 102, a cell arrangement section 103, an observation section 104, and an analysis section 105. In addition to these, a drug addition section 114 is preferably provided, to constitute a main section 100 (a section surrounded by a thick line) of the cell observation device 1000.

A cell sorting section 101 may be provided upstream of the cell introduction section 102.

The cell sorting section 101 discriminates cells placed therein by one or a plurality of parameters, and sortingly collects presets types of cells.

Examples of the parameter include intensity of fluorescence, size, form, and electrical properties of cells.

Examples of a specific device for use as the cell sorting section 101 include a FACS and a filter.

Note that the installation of the cell sorting section 101 is arbitrary, and cells may be preliminarily separated according to their types and may then be introduced into the cell introduction section 102.

The cell introduction section 102 has a channel through which the cells prepared in the cell sorting section 101 are introduced into the cell arrangement section 103.

A drug may be added to the cell introduction section 102, and different drugs may be added according to each of the kinds of cells.

The method for cell introduction in the cell introduction section 102 may be, for example, suction of cells from the side of the cell arrangement section 103 disposed downstream.

In addition, examples of the drug include carcinostatic agents such as molecular target drugs and immune check point inhibitors (anti-CTLA-4 antibody, anti-PD-1 antibody, anti-PD-Ll antibody, etc.), interleukins (IL-2, etc.), and interferons (IFN-γ, etc.).

The cell arrangement section 103 is provided with wells, and one or a plurality of kinds of cells introduced therein are mixed and arranged in the wells. The number of the cells is not particularly limited, and may be widely set to be several tens to several tens of thousands or more. Specific examples of the cells include suction wells and precipitation wells.

The drug can be introduced into the cell arrangement section 103, and the drug may be introduced directly into the cell arrangement section 103 without passing through the cell introduction section 101.

The number of the cells placed into the well may be one or plural.

Besides, the cell arrangement section may be controlled as to cell environments such as oxygen, temperature and pH by a cell environment control section 113 which will be described later.

The plurality of cells placed in each well of the cell arrangement section 103 contact each other, and some event or events occur. Examples of the event include movement of cells, variation in moving speed of cells, variation in form of cells, secretion release of cells, predation of cells, cell death, cell growth, variation in intensity of fluorescence from cells, variation in distance between cells, generation of intracellular granules, and localization of intracellular granules.

For instance, when immune cells and cancer cells are placed in each well, the immune cells and cancer cells contact each other to generate cytotoxicity, resulting in death of the cancer cells.

Examples of the immune cells include natural killer (NK) cells, T cells, microphage, dendritic cells, neutrophils, acidophils, basophils, and killer T cells. One or a plurality of these kinds may be selected.

The observation section 104 temporally observes the cells arranged in the cell arrangement section 103. In the observation, the event arising from the cell contact can be traced.

The method for observation is not particularly limited, and the observation may be performed by utilizing, for example, a bright field image, a phase difference image, a fluorescent image, or a refractive index image. In the case where fluorescent strain is utilized, for example, cancer cells may be preliminarily subjected to gene recombination such that expression of fluorescent protein such as GFP will be obtained, whereby the process in which the cancer cells are injured or preyed on by immune cells to reach death can be traced on a fluorescence basis. The observation is conducted continuously, for example, for several minutes to several days.

In addition, examples of a specific device for use at the observation section 103 include a microscope and an image sensor such as a CMOS.

The analysis section 105 analyzes data obtained by the observation section, for example, image data. The data are images representing a movement, contact, variation in form, secretion release, predation, death and growth of the cells or the like, and these can be turned into numerical values and/or a graph.

Examples of a specific device for use as the analysis section 105 include a personal computer and a program for analysis.

In addition, the analysis may be performed as to the observed event on a real-time basis, or may be conducted after the observed event is acquired and stored as data.

An example of analysis will be described later.

Besides, the cell observation device of the present technology may have a data storage section 107.

The data storage section 107 stores the observation data and the analysis data.

Examples of a specific device for use as the data storage section 107 include a server and a memory disc.

Further, a database 115 may be provided downstream of the data storage section 107, to accumulate various data, such that search, extraction and the like of past data can be performed.

Alternatively, the cell observation device of the present technology may have a comparison section 116 downstream of the analysis section 105. The comparison section 116 is capable of, for example, comparison between data in the analysis section 105 with control data or past analysis data.

Examples of a specific device for use as the comparison section 116 include a database reference personal computer and a program.

In addition, a display section 106 may be provided.

The display section 106 displays the observation data and the analysis data.

Examples of a specific device for used as the display section 106 include a PC monitor.

When the observation of the cell contact is finished, the cells in the well of the cell arrangement section 103 can be taken out. For example, a cell taking-out section 108 is provided, and the cell or cells specified in the analysis are taken out.

Examples of a specific device for used as the cell taking-out section 108 include a pipette.

The cells taken out are held by a cell holding section 109. A plurality of cell holding sections 109 may be provided according to, for example, the kinds of cells. The cell holding section 109 is controlled as to oxygen, temperature, pH or the like by the cell environment control section 113 according to the cells.

Besides, the cell holding section 109 can be added a reagent from the drug addition section 114.

Alternatively, a pretreatment for gene analysis at a gene analysis section 110 disposed downstream can be performed.

Examples of a specific device for use as the cell holding section 109 include a 96-well plate.

The gene analysis section 110 analyzes cells held in the cell holding section 109.

Examples of a specific device for use as the gene analysis section 110 include a DNA sequencer and an RNA sequencer.

In addition, the cell observation device of the present technology may have a channel priming section 111 and a chip disposing section 112.

The channel priming section 111 prepares a channel chip. The channel priming section 111 is capable of, for example, an operation for removing bubbles or a coatability improving operation (e.g., permitting ethanol to flow and then washing with water or a buffer).

The chip disposing section 112 correctly attaches the channel chip, which has been washed in the channel priming section 111, in such a manner that the cells are arranged in the cell arrangement section.

Further, the cell environment control section 113 may be provided, as aforementioned. The cell environment control section 113 adjusts or controls the environment for the cells placed in the cell arrangement section 103 and the cell holding section 109.

Examples of a specific device for use as the cell environment control section 113 include a CO₂-O₂ regulator, a temperature control device, and a culture medium exchange device.

In addition, different drugs or reagents can be placed in the drug addition section 114, the cell introduction section 102, the cell arrangement section 103, and the cell holding section 109. A plurality of drugs or reagents may be placed in the same section.

Examples of a specific device for use as the drug addition section 114 include a sampler (pipette) and a microchannel.

Specific examples of the drug or reagent include cell activators such as cytokine, drugs for examining the effectiveness on cancer cells injured or preyed on by immune cells, cancer antigens, or cancer cells, carcinostatic agents, staining reagents, cell dispersion reagents, viruses, bacteria, fungi, parasites, and allergens.

### 1-2. Example of cell observation and analysis

An example of a series of flow from fractionating target cells from a sample to observation and analysis of the target cells by the cell observation device of the present technology is depicted in FIG. 2.

A sample includes a plurality of kinds of cells, for example, regulatory T cells (Treg) 201, marrow-derived suppressor cells (MDSC) 202, dendritic cells (DC) 203, cytotoxic T cells (CTL) 204, and cancer cells 205.

The sample is sorted by a flow cytometer 2000, and kinds of cells are fractionated on a kind basis (for example, cytotoxic T cells 204, and cancer cells 205).

Here, the cancer cells may be subjected to specification of the kind of cancer cells by use of antibodies 206 specific to the kinds of the cancer cells.

The cytotoxic T cells 204 and the cancer cells 205 fractionated are taken from the cell introduction section 102 and arranged in wells 1031 in the cell arrangement section 103.

Thereafter, temporal observation is conducted; the observation may be conducted on the basis of each wall 1031, or may be conducted over the whole of the cell arrangement section 103. Alternatively, the cell arrangement section 103 may be partitioned into some parts, different kinds of cells may be introduced into different parts, and observation may be conducted.

In the well 1031, an attack on the cancer cells 205 by the cytotoxic T cells 204 and death of the cancer cells are generated. A series of the events are observed by a CMOS or the like, and data are recorded.

FIG. 3 depicts a flow chart of a process from cell sorting to cell reaction analysis according to the present technology.

First, a cell sample (S301) is served to a flow cytometer, to perform cell sorting (S302) on the basis of the kind of cells.

The cells on one side are introduced as they are into the cell introduction section 102 of the cell observation device 1000 according to the present technology (S303).

The cells on the other side are treated with a drug supplied from the drug addition section 114 of the cell observation device 1000 (S304), and are introduced into the cell introduction section 102 (S305).

A desired number of the cells are arranged into each well of the cell arrangement section 103 from the cell introduction section 102 (S306).

The cells arranged in the cell arrangement section 103 undergo a reaction, secretion release, predation, cell death or the like arising from cell contact, and these events are temporally observed by the observation section 104. For example, the events are observed by a phase difference microscope, a fluorescent microscope, a CCD, a CMOS or the like (S307), and can be recorded as data.

### 1-3. Data analysis

For example, data obtained from a reaction arising from cell contact are analyzed by the analysis section 105 (S308).

An example of an operation of the analysis section 105 is depicted in FIG. 4.

In the case of analyzing fluorescent image data (S401), image processing of the data is conducted (S402). The image processing is, for example, contrast adjustment, image threshold segmentation, noise treatment, or border exclusion.

Next, a cell identification treatment is performed (S403). In the cell identification treatment, the differences in the kind of cells are identified, and the cells are separated on a kind basis. For instance, the cells can be identified and separated by color, size, texture such as the feel of material, or the like. By the cell identification treatment, position information associated with the detected cells can also be determined on the basis of a predetermined interval in the past time and on a kind basis when the treatment is finished (S404), and the position information can be one of data.

Subsequently, an event detection treatment is conducted (S405). The event detection treatment detects an event arising from an interaction of cells. Examples of the event include a change in color of cells (apoptosis reaction reagent), a change in form of cells (apoptosis form change), co-localization of two kinds of cells (when immune cells depict coloring in green, red in which cancer cells depict coloring is contained in the green of immune cells in a certain proportion), and contact (the boundary between the immune cell and the cancer cell is at a distance equal to or smaller than a predetermined value). These events are detected at predetermined time intervals. Since these events are observed temporally, the definition or determination of the events may be somewhat obscure, and the determination times for different events may overlap with each other.

In addition, these events may be detected and calculated on a cell basis, or may be detected and calculated collectively on the basis of the cells introduced into the well of the cell arrangement section 103 (S406). For example, as depicted in FIG. 5 (simulated graph), the event can be represented by a cumulative graph (207) in which the cancer cell death rate for all wells is traced with the lapse of time.

When the event is detected (S407), region of interests (ROI) in regard of the cells for which the event has been detected is formed by an image analyzer having an ROI function (S408), for the purpose of extracting a portion to be further analyzed from the image data, quantitative determination of fluorescence, or the like. The ROI may be formed in a size coinciding with the size of the cells, or may be formed in an arbitrary size in relation to the size of the cells. In addition, the shape of the ROI is not particularly limited.

Next, movement of the cells in the ROI formed is analyzed before and after the event, and an index of the movement is calculated and evaluated (S409). Examples of the index of the movement of the cells include movement evaluations by cumulative moving distance between ROI frames, and automatic multi-variable image analysis (MVA). For example, as depicted in FIG. 6 (simulated graph), the activeness of variation in movement of cytotoxic T cells in each well (S410) can be represented by an index be represented as a graph (208).

Other examples of representation of data include distribution information of movement of cells before and after a specific event, image representation of tracing of movement on a cell basis, the number of cancer cells brought to death per immune cell, and representation of lapse of time as to a specific event.

### <2. Method for evaluating activity level of immune cells>

### 2-1. Evaluation by cytotoxicity assay

Where immune cells (NK cells, T cells, macrophage, dendritic cells, or the like) are brought into contact with cancer cells to cause injury of the cancer cells by use of the cell observation device of the present technology, as aforementioned, it is thereby possible to perform evaluation of activity of the immune cells in that instance, evaluation of affinity-for-injury relationship between immune cells and cancer cells, or the like.

Specifically, for example, evaluation can be performed by the following calculation methods.
Calculation of cancer cell survival rate: (Number of live cancer cells after cell contact)/(Total number of cancer cells)
Calculation of cancer cell fatality rate: (Number of cancer cells brought to death after cell contact)/(Total number of cancer cells)
Calculation of immune cell survival rate: (Number of live immune cells after cell contact)/(Total number of immune cells)
Calculation of immune cell fatality rate: (Number of immune cells brought to death after cell contact)/(Total number of immune cells)

Further, the efficiency of cell contact assay can be represented by:
(Number of cells brought into contact)/(Number of non-contact cells), or (Number of cells brought into contact)/(Total number of cells).

In the case of the above-mentioned calculation methods, it is preferable to take a negative control as a background.

Here, the negative control refers to cells that do not include the immune cells or are negative in the case of cancer cell life/death determination, or cells that do not include cancer cells or are negative in the case of immune cell life/death determination, or molecules that do not include a carcinostatic agent or do not undergo a reaction in the case of carcinostatic agent evaluation.

Note that the number of cells at a time point before the start of the reaction arising from cell contact is made to be 100% alive.

Note that rates of life and death can be calculated with limitation to the cancer cells brought to cell contact, by using

Calculation of rate of cancer cell death due to cell contact: (Number of dead cancer cells after cell contact)/(Total number of cancer cells brought to cell contact), and

Calculation of rate of cancer cell life after cell contact: (Number of live cancer cells after cell contact)/(Total number of cancer cells brought to cell contact). In this case, life and death of cancer cells due to immune cells can be calculated, without taking a background.

Identification of immune cells and cancer cells can be performed by, for example, form, size, fluorescent or the like staining, or movement.

In addition, determination of cell contact can be conducted by, for example, contact time, stop of movement of cells (moving speed), localization of granules, or adjacent arrangement of cells having different fluorescent dyes (fluorescent staining).

Determination of cell death can be performed by, for example, a change in form of cells, or augmentation of signal of cell death determination dye (fluorescent staining).

Note that the evaluation can also be performed by observation and analysis of cells on a real-time basis.

### 2-2. Evaluation by secretion release assay

In secretion release assay, the presence or absence of release of a secretion from cells attendant on cell contact is evaluated.

The determination of the cell contact is the same as aforementioned.

The determination of secretion release can be conducted by, for example, a change in form of cells, formation of granules, immunoassay of secretions, or existing assay of calcium or the like.

### 2-3. Evaluation by antibody-dependent cellular cytotoxicity

The activity level of immune cells can be evaluated by applying Antibody-Dependent Cellular Cytotoxicity (ADCC).

For example, injury on cancer cells by immune cells (NK cells, T cells, macrophage, dendritic cells, or the like) through an antibody is observed.

The evaluation of antibody-dependent cellular cytotoxicity can be judged by, for example, evaluation of binding of cancer cell and antibody, evaluations of binding and activation of antibody and immune cell, evaluation of injury on cancer cells by immune cells, or the like.

In addition, not only death of cancer cells but also a trace of an interaction between immune cells and cancer cells through an antibody may be observed.

For example, the mechanism of effectiveness of a carcinostatic agent can be examined.

Specifically, it is sufficient that contact time of NK cells and cancer cells is measured, observed, and affinity between an Fc portion of an antibody and FcγIII being in expression on the NK cells is observed.

Besides, it is sufficient to observe expression induction of protein such as perforin or granzyme in cytoplasm of NK cells, or formation of granules.

Further, intrusion of protein such as perforin into target cells may be observed.

Furthermore, cell death induction or apoptosis non-induction as to cancer cells may also be observed.

### 2-4. Application example 1

It is also possible to evaluate a carcinostatic agent at the time of drug development by applying the present technology.

For example, a candidate carcinostatic agent is administered to cancer cells arranged in a well, and immune cells (for example, NK cells, cytotoxic T cells) are introduced into the same cell. The resulting event is observed and analyzed by the present technology, thereby judging life or death of the cancer cells.

In addition, in the case of immunotherapy, by growing immune cells, administering the grown immune cells to cancer cells, and observing a change in the cancer cells, it is possible to check the effect. Further, the present technology may be applied to a cancer immune check-point inhibitor.

### 2-5. Application example 2

It is also possible to select T cell receptor (TCR) by a reaction between cancer cells and tumor infiltrating lymphocytes (TIL), by applying the present technology.

First, TIL are isolated from the cancer cells, or T cells are isolated from blood.

Next, the isolated TIL or T cells are treated with IL-2 or IFN-γ or the like.

On the other hand, the cancer cells are treated with an immune check-point inhibitor (for example, Opdivo (registered trademark)).

The treated TIL or T cells and the treated cancer cells are brought into contact with each other, followed by observation and analysis by the present technology, whereby an individual of the TIL or T cells depicting a specific reaction to a cancer antigen of the cancer cell is identified.

The identified cell is isolated, and sequence analysis of DNA, RNA and the like possessed by the cell is conducted, to specify the TCR.

By application of the present technology, the above-mentioned application examples and the like can be performed even where the number of immune cells such as TIL or T cells is small, and smaller amounts of drugs and reagents are required to be used.

### <3. Method for controlling quality of immune cells>

In recent years, attention has been paid to immunotherapy of cancers, and it is necessary to grow immune cells outside the living body, and, after activation, to administer the grown and activated immune cells into the body.

The present technology is applicable also to evaluate and control quality of the grown immune cells on a single cell basis, instead of a bulk basis.

Specifically, the method for controlling quality of immune cells of the present technology can be carried out by:
bringing a plurality of cancer cells to a single immune cell;
examining the number of cancer cells injured or preyed on by the single immune cell; and
excluding the single immune cell in the case where the single immune cell has preyed on a number of less than a predetermined number of cancer cells or in the case where the single immune cell has injured or preyed on a number of more than a predetermined number of cancer cells, within a predetermined time.

Here, a simulated example of data obtained when the evaluation of activity level of an immune cell isolated from a living body is conducted using the present technology will be depicted.

In the case of evaluating activity level of immune cells derived from a living body, a variety of cell groups including various kinds of immune cells are provided. In that case, the number of cells injured differs depending on the compatibility between the individual immune cells and the target cells.

In view of this, for example, an ID number is given to each immune cell, one immune cell is arranged in a well, further approximately 10 cancer cells are arranged in the same cell, and how many cancer cells are injured by the immune cell is observed and analyzed by use of the present technology; in this case, it is assumed that the data set forth in Table 1 below are obtained.

**[Table 1]**

| <Assumed data> | | | | | | |
|---|---|---|---|---|---|---|
| True state | Rate of cells depicting high cytotoxicity | | | Many | None | Few |
| | | Cell ID No. | reference data | Sample 1 | Sample 2 | Sample3 |
| | Number of cells injured | 1 | 5 | 4 | 0 | 1 |
| | | 2 | 4 | 6 | 1 | 0 |
| | | 3 | 6 | 5 | 0 | 0 |
| | | 4 | 4 | 5 | 0 | 1 |
| | | 5 | 5 | 6 | 0 | 6 |
| | | 6 | 6 | 6 | 1 | 0 |
| | | 7 | 5 | 4 | 0 | 5 |
| | | 8 | 6 | 5 | 0 | 0 |
| | | 9 | 4 | 5 | 0 | 1 |
| | | 10 | 5 | 4 | 0 | 0 |
| | | Average | 5 | 5 | 0.2 | 1.4 |
| | | S.D. | 0.8 | 0.8 | 0.4 | 2.2 |
| | | CV% | 16.3 | 16.3 | 210.8 | 158.7 |
| | | not more than 3 | 0 | 0 | 10 | 8 |
| | Number of cancer cells injured | 4 to 6 | 10 | 10 | 0 | 2 |
| | | not less than 7 | 0 | 0 | 0 | 0 |
| | Presence/absence of immune cells of high compatibility | | | Present | Absent | Present |

At the time of controlling the quality of immune cells, it is undesirable that the activity of the immune cells is too strong or is too weak. The immune cells with too high an activity may cause such a symptom as autoimmune disease, for example, when the immune cells are administered to a subject. On the other hand, the immune cells with too low an activity fails to depict a sufficient function as immune cell when the immune cells are administered to a subject.

In the simulated example of Table 1 above, for example, a single immune cell depicting a number of cells injured in the range of 4 to 6 can be selected.

The existing quality control has been made on a bulk basis; therefore, as indicated in Table 1, even where the immune cells depicting a number of cells injured in the range of 4 to 6 are present, these immune cells cannot be excluded. In other words, even when a good activity is depicted on a bulk basis as a whole, the individual immune cells have included immune cells with high activity and immune cells with low activity in a mixed state.

The present technology is applicable also to quality control during production of immune cells.

In the production of immune cells, it is necessary to appropriately control the storage state and the like, and to confirm that the quality of the cells as a whole of the cells produced is high.

On the other hand, there may be cases where the activation of cells is nonuniform in the production process of immune cells, and, therefore, it is necessary to confirm that the quality is high also on a single cell basis.

According to the present technology, it is possible to control the quality of cells, by performing cell evaluation on a single cell basis.

Here, a simulated example of data obtained when the evaluation of activity level of immune cells for quality control during production of immune cells is conducted using the present technology is depicted in Table 2.

**[Table 2]**

| <Assumed data> | | | | | | | |
|---|---|---|---|---|---|---|---|
| True state | Cell storage state | | | High | Low | High | High |
| | Cell production state | | | High | High | Low | Low |
| Calculated value | Overall cell quality | | | High | Low | Low | High |
| | Individual cell quality | | | High | Low | High & low | Extremely high & extremely low |

| | | Cell ID No. | reference data | Sample 1 | Sample 2 | Sample 3 | Sample 4 |
|---|---|---|---|---|---|---|---|
| | Number of cells injured | 1 | 5 | 4 | 2 | 5 | 8 |
| | | 2 | 4 | 6 | 2 | 1 | 1 |
| | | 3 | 6 | 5 | 1 | 1 | 0 |
| | | 4 | 4 | 5 | 2 | 6 | 7 |
| | | 5 | 5 | 6 | 2 | 6 | 9 |
| | | 6 | 6 | 6 | 3 | 1 | 7 |
| | | 7 | 5 | 4 | 2 | 1 | 0 |
| | | 8 | 6 | 5 | 3 | 2 | 1 |
| | | 9 | 4 | 5 | 1 | 1 | 8 |
| | | 10 | 5 | 4 | 2 | 6 | 9 |
| | | Average | 5 | 5 | 2 | 3 | 5 |
| | | S.D. | 0.8 | 0.8 | 0.7 | 2.4 | 3.9 |
| | | CV% | 16.3 | 16.3 | 33.3 | 80.1 | 78.9 |
| | | not more than 3 | 0 | 0 | 10 | 6 | 4 |
| | | 4 to 6 | 10 | 10 | 0 | 4 | 0 |
| | | not less than 7 | 0 | 0 | 0 | 0 | 6 |
| | Quality determination | | Hiqh | Low | Low | Low | Low |

As aforementioned, in the existing quality control on a bulk basis, immune cells including those of extremely high activity and those of extremely low activity in a mixed state cannot be excluded. According to the present technology, on the other hand, a sample depicting a number of cells injured in the range of 4 to 6 can be selected.

Note that quality control immediately before administration of immune cells to a patient can also be conducted in a similar way.

### [Reference Signs List]

100 Main section
101 Cell sorting section
102 Cell introduction section
103 Cell arrangement section
104 Observation section
105 Analysis section
106 Display section
107 Data storage section
108 Cell taking-out section
109 Cell holding section
110 Gene analysis section
111 Channel priming section
112 Chip disposing section
113 Cell environment control section
114 Drug addition section
115 Database
201 Regulatory T cells
202 Marrow-derived suppressor cells
203 Dendritic cells
204 Cytotoxic T cells
205 Cancer cells
206 Antibody
207 Cumulative graph in which cancer cell death rate for all wells is traced with lapse of time
208 Graph of variation in movement of cytotoxic T cells
1000 Cell observation device
1031 Well
2000 Flow cytometer

## Claims

1. A method for evaluating activity level of immune cells, comprising:
bringing a plurality of cancer cells into contact with a single immune cell; and
examining the number of cancer cells brought to death by the single immune cell within a predetermined time.

2. A cell observation device for carrying out a method according to claim 1, the apparatus comprising:
a cell introduction section;
a cell arrangement section comprising a plurality of wells;
an observation section for obtaining image data of cells within the cell arrangement section; and
an analysis section for analyzing the image data,
wherein the cell introduction section is configured to introduce immune cells and cancer cells into the cell arrangement section,
wherein one well of the cell arrangement section is arranged to contain a single immune cell and a plurality of cancer cells at an initial time,
the observation section is configured to make an observation of a temporal event resulting from cell contact in the one well of the cell arrangement section, and
the analysis section is configured to analyze the temporal event resulting from the cell contact to examine the number of the cancer cells brought to death by the single immune cell within a predetermined time of the initial time.

3. The cell observation device according to claim 2, comprising:
a drug addition section configured to add a drug to the cell introduction section and/or the cell arrangement section.

4. The cell observation device according to claim 2, wherein the temporal event arising from the cell contact is selected from a group including movement of cells, variation in moving speed of cells, variation in form of cells, secretion release of cells, predation of cells, cell death, cell growth, variation in intensity of fluorescence from cells, variation in distance between cells, generation of intracellular granules, and localization of intracellular granules.

5. The cell observation device according to claim 2, wherein the temporal event arising from the cell contact is observed by use of an image selected from a group including a bright field image, a phase difference image, a fluorescent image, and a refractive index image.

6. A method for controlling quality of immune cells, comprising:
bringing a plurality of cancer cells into contact with a single immune cell;
examining the number of cancer cells brought to death by the single immune cell; and
excluding the single immune cell in a case where the single immune cell has brought a number of less than a predetermined number of the cancer cells to death or in a case where the single immune cell has brought a number of more than a predetermined number of the cancer cells to death within a predetermined time.

## Patentansprüche

1. Verfahren zum Bewerten des Aktivitätsniveaus von Immunzellen, Folgendes umfassend:
Inkontaktbringung einer Vielzahl von Krebszellen mit einer einzelnen Immunzelle; und
Untersuchen der Anzahl von Krebszellen, die innerhalb einer vorbestimmten Zeit durch die einzelne Immunzelle zum Tod gebracht wurden.

2. Zellenbeobachtungsvorrichtung zum Ausführen eines Verfahrens gemäß Anspruch 1, wobei die Vorrichtung Folgendes umfasst:
einen Zelleneinführungsteil;
einen Zellenanordnungsteil, der eine Vielzahl von Wannen umfasst;
einen Beobachtungsteil zum Erhalten von Bilddaten von Zellen innerhalb des Zellenanordnungsteils; und
einen Analyseteil zum Analysieren der Bilddaten,
wobei der Zelleneinführungsteil dazu ausgelegt ist, Immunzellen und Krebszellen in den Zellenanordnungsteil einzuführen,
wobei eine Wanne des Zellenanordnungsteils dazu ausgebildet ist, eine einzelne Immunzelle und eine Vielzahl von Krebszellen zu einer Anfangszeit zu enthalten,
der Beobachtungsteil dazu ausgelegt ist, eine Beobachtung eines zeitlichen Ereignisses zu machen, das aus Zellenkontakt in der einen Wanne des Zellenanordnungsteils resultiert, und
der Analyseteil dazu ausgelegt ist, das aus dem Zellenkontakt resultierende zeitliche Ereignis zu analysieren, um die Anzahl der Krebszellen zu untersuchen, die innerhalb der Anfangszeit durch die einzelne Immunzelle zum Tod gebracht wurden.

3. Zellenbeobachtungsvorrichtung gemäß Anspruch 2, Folgendes umfassend:
einen Medikamentenzugabeteil, der dazu ausgelegt ist, ein Medikament zu dem Zelleneinführungsteil und/oder dem Zellenanordnungsteil zuzugeben.

4. Zellenbeobachtungsvorrichtung gemäß Anspruch 2,
wobei das aus dem Zellenkontakt entstehende zeitliche Ereignis von einer Gruppe ausgewählt wird, die Folgendes umfasst: Bewegung von Zellen, Variation in der Bewegungsgeschwindigkeit von Zellen, Variation in der Form von Zellen, Sekretionsfreigabe von Zellen, Prädation von Zellen, Zellentod, Zellenwachstum, Variation in der Intensität der Fluoreszenz von Zellen, Variation im Abstand zwischen Zellen, Erzeugung von intrazellulären Granula und Lokalisierung von intrazellulären Granula.

5. Zellenbeobachtungsvorrichtung gemäß Anspruch 2,
wobei das aus dem Zellenkontakt entstehende zeitliche Ereignis durch Verwendung eines Bilds beobachtet wird, das von einer Gruppe ausgewählt wird, die Folgendes umfasst: ein Hellfeldbild, ein Phasendifferenzbild, ein Fluoreszenzbild und ein Brechungsindexbild.

6. Verfahren zum Steuern der Qualität von Immunzellen, Folgendes umfassend:
Inkontaktbringung einer Vielzahl von Krebszellen mit einer einzelnen Immunzelle; und
Untersuchen der Anzahl von Krebszellen, die durch die einzelne Immunzelle zum Tod gebracht wurden; und
Ausschließen der einzelnen Immunzelle in einem Fall, in dem die einzelne Immunzelle eine Anzahl von weniger als einer vorbestimmten Anzahl der Krebszellen zum Tod gebracht hat, oder in einem Fall, in dem die einzelne Immunzelle eine Anzahl von mehr als einer vorbestimmten Anzahl der Krebszellen innerhalb einer vorbestimmten Zeit zum Tod gebracht hat.

## Revendications

1. Procédé permettant d'évaluer un niveau d'activité de cellules immunitaires, comprenant :
la mise en contact de multiples cellules cancéreuses avec une cellule immunitaire unique ; et
l'examen du nombre de cellules cancéreuses conduites à la mort par la cellule immunitaire unique dans un temps prédéfini.

2. Dispositif d'observation de cellules pour réaliser un procédé selon la revendication 1, l'appareil comprenant :
une section d'introduction de cellules ;
une section d'agencement de cellules comprenant une pluralité de puits ;
une section d'observation pour obtenir des données d'image de cellules dans la section d'agencement de cellules ; et
une section d'analyse pour analyser les données d'image,
dans lequel la section d'introduction de cellules est configurée pour introduire des cellules immunitaires et des cellules cancéreuses dans la section d'agencement de cellules,
dans lequel un puits de la section d'agencement de cellules est conçu pour contenir une cellule immunitaire unique et une pluralité de cellules cancéreuses à un moment initial,
la section d'observation est configurée pour faire une observation d'un événement temporel obtenu d'un contact cellulaire dans le puits de la section d'agencement de cellules, et
la section d'analyse est configurée pour analyser l'événement temporel obtenu du contact cellulaire pour examiner le nombre de cellules cancéreuses conduites à la mort par la cellule immunitaire unique dans un temps prédéfini du temps initial.

3. Dispositif d'observation de cellules selon la revendication 2, comprenant :
une section d'ajout de médicament configurée pour ajouter un médicament à la section d'introduction de cellules et/ou la section d'agencement de cellules.

4. Dispositif d'observation de cellules selon la revendication 2, dans lequel l'événement temporel se produisant à partir du contact cellulaire est choisi dans un groupe comprenant un mouvement des cellules, une variation de la vitesse de déplacement des cellules, une variation de forme des cellules, une libération de sécrétion des cellules, une prédation des cellules, une mort cellulaire, une croissance cellulaire, une variation de l'intensité de fluorescence provenant des cellules, une variation de la distance entre les cellules, une génération de granules intracellulaires et une localisation de granules intracellulaires.

5. Dispositif d'observation de cellules selon la revendication 2, dans lequel l'événement temporel se produisant à partir du contact cellulaire est observé au moyen d'une image choisie dans un groupe comprenant une image de champ lumineux, une image à différence de phase, une image fluorescente et une image à indice de réfraction.

6. Procédé permettant de contrôler la qualité de cellules immunitaires, comprenant :
la mise en contact de multiples cellules cancéreuses avec une cellule immunitaire unique ;
l'examen du nombre de cellules cancéreuses conduites à la mort par la cellule immunitaire unique ; et
l'exclusion de la cellule immunitaire unique dans un cas où la cellule immunitaire unique a conduit à la mort un nombre inférieur à un nombre prédéfini des cellules cancéreuses ou dans un cas où la cellule immunitaire unique a conduit à la mort un nombre supérieur à un nombre prédéfini des cellules cancéreuses dans un temps prédéfini.
